# EUROPEAN PATENT APPLICATION

(11) **EP 4 502 916 A1**
(43) Date of publication of application: **05.02.2025**
(21) Application number: 23778737.9
(22) Date of filing: 13.01.2023
(51) Int. Cl.: G06Q 50/10

(54) **EVALUATION METHOD, EVALUATION DEVICE, AND PROGRAM**

(30) Priority: 31.03.2022 JP 2022059709
(71) Applicant: Shimadzu Corporation, Kyoto-shi, Kyoto 604-8511 (JP)
(72) Inventor: MURATA, Koichi, Kyoto-shi, Kyoto 604-8511 (JP); FURUTA, Masafumi, Kyoto-shi, Kyoto 604-8511 (JP); URAOKA, Yasuyuki, Kyoto-shi, Kyoto 604-8511 (JP)
(74) Representative: Müller-Boré & Partner Patentanwälte PartG mbB
(86) International application number: PCT/JP2023/000831
(87) International publication number: WO 2023/188698

(57) **Abstract**

An evaluation method comprises: obtaining information about a task performed by a subject (step S10); obtaining biological information of the subject (step S12); determining a timing at which the biological information satisfies a predetermined condition (step S14); outputting a portion of the information about the task corresponding to the determined timing (step S24); and receiving an input of feedback on the task (step S26).

## Description

### TECHNICAL FIELD

The present invention relates to evaluating a subject's response through feedback from the subject.

### BACKGROUND ART

Conventionally, providers of goods or services have utilized feedback from subjects (e.g., customers of goods or services) to evaluate the subject's response to the goods or services. For obtaining feedback, for example, U.S. Publication No. 2002/0044687 (PTL 1) discloses a technique of electronically sending a sheet having feedback information and a customer's address written thereon to the customer, electronically processing the feedback information, and providing an incentive to the customer using the address. Further, various studies have been done on a technique of obtaining information on brain activity and evaluating the information as feedback (see NPLs 1, 2 and 3).

### CITATION LIST

### PATENT LITERATURE

PTL 1: U.S. Publication No. 2002/0044687

### NON PATENT LITERATURE

NPL 1: "KANSEI_Analyzer - making neuromarketing more common," [online], [searched on November 14, 2021], DENTSU SCIENCEJAM, <URL: https://www.dentsusciencejam.com/kansei-analyzer/>
NPL 2: "Guidance for Valance-Arousal_Analyzer," [online], [searched on November 14, 2021], DENTSU SCIENCEJAM, <URL: https://www.dentsusciencejam.com/VAA/>
NPL 3: "Neuromarketing; Procedure of Kansei Evaluation," [online], [searched on November 14, 2021], DENTSU SCIENCEJAM, <https://neu-brains.co.jp/service/neuro-marketing/flow/>

### SUMMARY OF INVENTION

### TECHNICAL PROBLEM

According to conventional art, a subject's response to goods or services can be objectively evaluated. In obtaining specific feedback from the subject, however, systematically there is room for improvement in general.

The present invention has been made under such circumstances, and contemplates a technique for obtaining more specific feedback from a subject in evaluating the subject's response.

### SOLUTION TO PROBLEM

In one aspect of the present disclosure, an evaluation method comprises: obtaining information about a task performed by a subject; obtaining biological information of the subject performing the task over time; determining a timing at which the biological information satisfies a predetermined condition; outputting a portion of the information about the task corresponding to the determined timing; and receiving an input of feedback on the task from the subject.

In another aspect of the present disclosure, an evaluation method comprises: obtaining information about a task performed by a subject; determining a timing at which an input of a marker is received from at least one of the subject performing the task and an observer observing the subject while the subject performs the task; outputting a portion of the information about the task corresponding to the determined timing; and receiving an input of feedback on the task from the subject.

In one aspect of the present disclosure, an evaluation apparatus comprises: an interface that obtains information about a task performed by a subject and biometric information of the subject; a memory that stores information that specifies a condition for a feature value in the biological information; and a processor that determines a timing at which the feature value in the biological information satisfies the condition, wherein the processor outputs a portion of the information about the task corresponding to the determined timing, and receives an input of feedback on the task.

In another aspect of the present disclosure, an evaluation apparatus comprises: an interface that obtains information about a task performed by a subject and biometric information of the subject; and a processor that determines a timing at which an input of a marker is received from at least one of the subject performing the task and an observer observing the subject while the subject performs the task, wherein the processor outputs a portion of the information about the task corresponding to the determined timing, and receives an input of feedback on the task.

In one aspect of the present disclosure, a program causes a computer to perform: obtaining information about a task performed by a subject; obtaining biological information of the subject; determining a timing at which the biological information satisfies a predetermined condition; providing a portion of the information about the task corresponding to the determined timing; and receiving an input of feedback on the task.

In another aspect of the present disclosure, a program is executed by a computer to cause the computer to perform: obtaining information about a task performed by a subject; determining a timing at which an input of a marker is received from at least one of the subject performing the task and an observer observing the subject while the subject performs the task; outputting a portion of the information about the task corresponding to the determined timing; and receiving an input of feedback on the task from the subject.

### ADVANTAGEOUS EFFECTS OF INVENTION

According to one aspect of the present disclosure, more specific feedback is obtained from a subject in evaluating a response of the subject.

### BRIEF DESCRIPTION OF DRAWINGS

Fig. 1 generally shows an evaluation system including an evaluation apparatus according to the present disclosure.
Fig. 2 shows an example of a scene in which a subject 200 inputs feedback on a task in the evaluation system.
Fig. 3 is a diagram schematically showing a block configuration of the evaluation system.
Fig. 4 is a diagram for illustrating attachment sites for a first myoelectric potential sensor 11 and a second myoelectric potential sensor 12.
Fig. 5 generally represents an example of processing performed in evaluation system 100.
Fig. 6 is a flowchart of a process performed by a computer 90 to obtain feedback from subject 200 performing a task.
Fig. 7 is a diagram showing an example of a screen for input.
Fig. 8 is a diagram showing another example of the screen for input.

### DESCRIPTION OF EMBODIMENTS

Hereinafter, an embodiment of the present disclosure will be described in detail with reference to the accompanying drawings. In the figures, identical or equivalent components are identically denoted and will not be described repeatedly.

### [Overview of Evaluation Method]

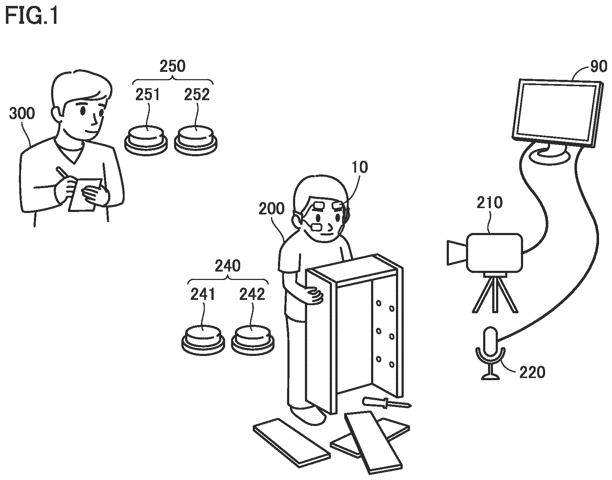

Fig. 1 generally shows an evaluation system including an evaluation apparatus according to the present disclosure. In the evaluation system, a computer 90 is an example of the evaluation apparatus.

A subject 200 performs a task to input feedback. In the example of Fig. 1, subject 200 assembles a shelf as an example of the task.

Computer 90 obtains information about the task performed by subject 200. In the example of Fig. 1, as one example of the information about the task, an image of a space in which subject 200 is performing the task (a bird's-eye view image) is captured with a camera 210 and transmitted to computer 90. As one example of the information about the task, audio in the space may be obtained with a microphone 220 and transmitted to computer 90.

Computer 90 also obtains biological information of subject 200. In the example of Fig. 1, a myoelectric potential signal of subject 200 is obtained as an example of the biological information.

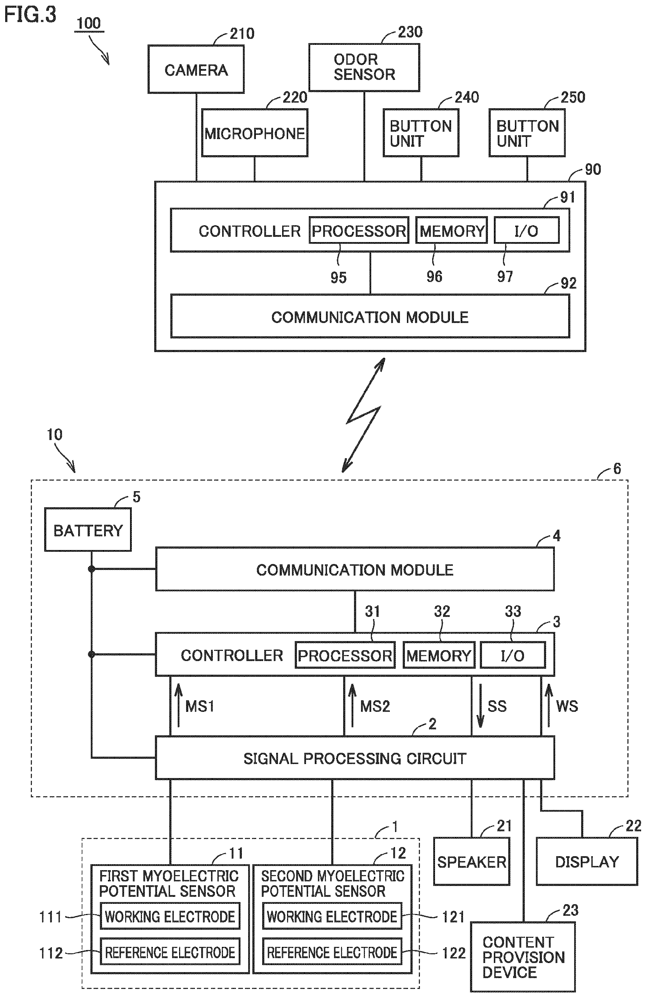

More specifically, computer 90 obtains the myoelectric potential signal of subject 200 via user device 10. Subject 200 wears user device 10. User device 10 includes one or more myoelectric potential sensors (a first myoelectric potential sensor 11 and a second myoelectric potential sensor 12, which will be described hereinafter with reference to Fig. 3 and the like.). The myoelectric potential signal is obtained by the one or more myoelectric potential sensors.

Computer 90 determines a timing at which the feature value of the biological information satisfies a predetermined condition, and provides to subject 200 a portion of the information about the task corresponding to the determined timing. As such a timing as described above, computer 90 may determine a timing at which one or more buttons included in a button unit 240 or a button unit 250 are operated. Button unit 240 includes two buttons 241 and 242 operated by subject 200. Button unit 250 includes two buttons 251 and 252 operated by an observer 300.

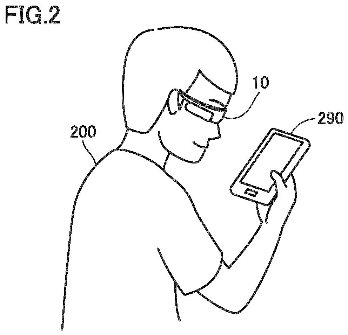

Fig. 2 shows an example of a scene in which subject 200 inputs feedback on a task in the evaluation system. In the example of Fig. 2, subject 200 obtains the above-described portion of the information about the task that is provided from computer 90 by a smartphone 290. In one implementation, the portion is a motion video captured with camera 210 at a timing determined as described above. Subject 200 inputs feedback on the task while confirming the portion with smartphone 290, and sends the feedback to computer 90.

### [Block Configuration]

Fig. 3 is a diagram schematically showing a block configuration of the evaluation system. As shown in Fig. 3, evaluation system 100 includes user device 10 and computer 90. In one implementation, user device 10 may include a wearable terminal worn by a subject. Computer 90 may be installed in an environment surrounding the subject. User device 10 and computer 90 are configured to be capable of bidirectional communication. Hereinafter, each configuration will be described.

### <User Device 10>

User device 10 includes a sensor unit 1, a signal processing circuit 2, a controller 3, a communication module 4, a battery 5, and a housing 6. Housing 6 houses signal processing circuit 2, controller 3, communication module 4, and battery 5 therein.

Sensor unit 1 includes a first myoelectric potential sensor 11 and a second myoelectric potential sensor 12. First myoelectric potential sensor 11 and/or second myoelectric potential sensor 12 are/is attached to the face of the subject to sense a myoelectric potential signal of a site to which the sensor/sensors is/are attached.

The myoelectric potential signal means a weak electrical signal generated when a muscle is moved. In the present disclosure, a subject's "face" is not limited to the facial region (a front or side surface of the face), and may include the neck of the subject. For example, sensor unit 1 may be attached to the throat of the subject to sense a change in myoelectric potential accompanying a swallowing action of the subject.

Sensor unit 1 may include a plurality of sensors. The plurality of sensors may be attached to different types of muscles. The types of muscles can be identified by sites at which the sensors are attached. In the first embodiment, when a type of muscle is identified, there is no need to identify the muscle in composition or structure. When the sensors are attached to different sites, different types of myoelectric potential signals can be obtained.

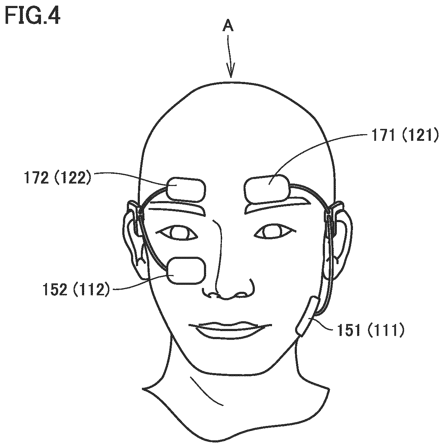

Fig. 4 is a diagram for illustrating attachment sites for first myoelectric potential sensor 11 and second myoelectric potential sensor 12. With reference to Fig. 4, one aspect in configuration and usage of first myoelectric potential sensor 11 and second myoelectric potential sensor 12 will be described.

First myoelectric potential sensor 11 is attached to the cheeks of the subject. First myoelectric potential sensor 11 includes a working electrode 111 and a reference electrode 112. Working electrode 111 is incorporated in a pad 151, and reference electrode 112 is incorporated in a pad 152.

First myoelectric potential sensor 11 senses a myoelectric potential signal of muscles in vicinities of the cheeks as pad 151 is attached directly on a laughing muscle and pad 152 is attached directly on a zygomaticus major muscle. More specifically, first myoelectric potential sensor 11 senses a potential of working electrode 111 with reference to a potential of reference electrode 112 as a myoelectric potential signal of a muscle in a vicinity of a cheek. Pad 151 and pad 152 (or working electrode 111 and reference electrode 112) may be attached to sites slightly shifted from the laughing muscle and the zygomaticus major muscle, respectively, insofar as the sites are in the vicinities of the muscles. First myoelectric potential sensor 11 outputs the myoelectric potential signals to signal processing circuit 2 of user device 10 as myoelectric potential signals indicating activities of facial muscles of the portions of the cheeks.

Second myoelectric potential sensor 12 is attached to the eyebrows of the subject. Second myoelectric potential sensor 12 includes a working electrode 121 and a reference electrode 122. Working electrode 121 is incorporated in a pad 171, and reference electrode 122 is incorporated in a pad 172.

Second myoelectric potential sensor 12 senses a myoelectric potential signal of a muscle in a vicinity of an eyebrow (for example, a corrugator supercilii muscle) as pad 171 and pad 172 are attached immediately above corrugator supercilii muscles. More specifically, second myoelectric potential sensor 12 senses a potential of working electrode 121 with reference to a potential of reference electrode 122 as a myoelectric potential signal of a muscle in a vicinity of an eyebrow. Pad 171 and pad 172 (or working electrode 121 and reference electrode 122) may be attached to sites slightly shifted from immediately above the corrugator supercilii muscles insofar as the sites are in the vicinities of the corrugator supercilii muscles. Second myoelectric potential sensor 12 outputs the myoelectric potential signals to signal processing circuit 2 as myoelectric potential signals indicating activities of the corrugator supercilii muscles.

In the example of Fig. 4, the laughing, zygomaticus major, and corrugator supercilii muscles are examples of facial muscles. More specifically, the laughing and zygomaticus major muscles are examples of muscles in a vicinity of a cheek. The corrugator supercilii muscle is an example of a muscle in a vicinity of an eyebrow.

Referring again to Fig. 3, signal processing circuit 2 applies prescribed signal processing (noise removal, rectification, amplification, digitization, etc.) to each myoelectric potential signal obtained from sensor unit 1 and outputs each processed signal to controller 3. Although not shown in the figure, signal processing circuit 2 includes a filter, an amplifier, and an A/D converter.

In Fig. 3, a signal received from first myoelectric potential sensor 11 and processed by signal processing circuit 2 is referred to as a "myoelectric potential signal MS1," and a signal received from second myoelectric potential sensor 12 and processed by signal processing circuit 2 is referred to as a "myoelectric potential signal MS2".

Controller 3 is a computing device including a processor 31, a memory 32, and an input/output port 33. Processor 31 is implemented for example by a CPU (a central processing unit). Memory 32 is implemented for example by a ROM (read only memory) and a RAM (random access memory). Input/output port 33 is an interface in controller 3 for inputting/outputting data. Controller 3 performs a computing process for evaluating an expression of a subject based on myoelectric potential signals MS1 and MS2.

Communication module 4 causes user device 10 to communicate with an external device, and is implemented for example by a communication device conforming to a short-range wireless communication standard. Controller 3 controls communication module 4 to control communication of information between user device 10 and outside (such as computer 90).

Battery 5 is a secondary battery such as a lithium ion secondary battery. Battery 5 supplies an operating voltage to each component in user device 10.

User device 10 further includes a speaker 21 and a display 22. Controller 3 outputs a control signal (a "signal SS" in Fig. 1) to speaker 21 and display 22 to provide an audio output through speaker 21.

User device 10 further includes a content provision device 23. Content provision device 23 is a device that provides contents. In one implementation, content provision device 23 may provide a taste to subject 200. Such a device is configured to allow the subject to for example perceive five tastes, that is, sweet taste, salty taste, acidic taste, bitter taste, and umami taste, electrically, and to vary a ratio at which these tastes are perceived to adjust a taste to be provided. More specifically, such a device, as known as a Norimaki Synthesizer (https://research.miyashita.com/papers/142/paper.pdf), incorporates 5 types of gels containing electrolytes that cause the subject to perceive the above 5 tastes, respectively, and adjusts an amount of electricity applied to the 5 types of gels to adjust a taste to be provided. In one implementation, content provision device 23 may provide a feel to subject 200. Such a device controls a state of a surface of a given object to adjust a tactile feel to be provided. In one implementation, content provision device 23 may provide an odor to subject 200. Such a device adjusts a content of each of one or more types of substances contained in supplied air to adjust an odor to be provided.

In evaluation system 100, information is obtained about a task performed by subject 200. And a portion of the obtained information corresponding to a timing at which biological information of subject 200 satisfies a predetermined condition is provided to subject 200.

The information about the task may be an image. In one example, a space in which the task is performed is imaged as the information about the task. In another example, an image provided to subject 200 while the task is performed is recorded as the information about the task. In that case, an image is provided to subject 200 as the above-described portion. The image is provided via display 22 (or a display device other than user device 10).

The information about the task may be audio. In one example, audio in the space in which the task is performed is recorded as the information about the task. In another example, audio provided to subject 200 while the task is performed is recorded as the information about the task. In that case, audio is provided to subject 200 as the above-described portion. The audio is provided via speaker 21 (or a speaker other than user device 10).

The information about the task may be information specifying an odor. In one example, as the information about the task, an odor in the space in which the task is performed is recorded for example as a composition of air. In another example, as the information about the task, an odor provided to subject 200 while the task is performed is recorded for example as a composition of air. In yet another example is recorded a substance determined from an image and/or audio provided to subject 200 while the task is performed and/or an image and/or audio recorded in the space. In that case, subject 200 is provided with a reproduced odor based on the determined information (e.g., a composition or air or a substance) as the above-described portion. For example, when "peach," a name of a substance, is determined from audio recorded in the space, an aroma of a peach may be reproduced and provided. The aroma is provided via content provision device 23.

The information about the task may be information specifying a feel. In one example, a feel (a state of a surface of an object) provided to subject 200 while the task is performed is recorded as the information about the task. In another example is recorded a substance determined from an image and/or audio provided to subject 200 while the task is performed and/or an image and/or audio recorded in the space. In that case, subject 200 is provided with a reproduced feel based on the determined information as the above-described portion. For example, when "peach," a name of a substance, is determined from audio recorded in the space, a predetermined surface state may be reproduced and provided as a feel of a surface of a peach. The feel is provided via content provision device 23.

The information about the task may be information specifying a taste. In one example, a taste provided to subject 200 while the task is performed (e.g., a name of a food product, information specifying a composition of a food product, etc.) is recorded as the information about the task. In another example is recorded a substance determined from an image and/or audio provided to subject 200 while the task is performed and/or an image and/or audio recorded in the space. In that case, a taste reproduced based on the determined information is provided to subject 200 as the above-described portion. For example, when "peach," a name of a substance, is determined from audio recorded in the space, a taste (a combination of the five tastes of sweet taste, salty taste, acidic taste, bitter taste, and umami taste) predetermined as a taste of a peach may be reproduced and provided. The taste is provided via content provision device 23.

The information about the task may be a combination of two or more of the above-described five types of information (image, audio, odor, feel, and taste). Content provision device 23 may include units each capable of reproducing information of a respective one of two or more types of odor, feel, and taste.

### <Computer 90>

Computer 90 is, for example, a personal computer (PC) or a server. Computer 90 communicates with user device 10 via a communication module (not shown) and receives a signal indicating a computation result of controller 3. Computer 90 includes a controller 91 and a communication module 92.

As well as controller 3, controller 91 includes a processor 95, a memory 96, and an input/output port 97, and executes a variety of computing processes. As well as communication module 4, communication module 92 is implemented for example by a communication device conforming to a short-range wireless communication standard. That is, communication module 92 causes computer 90 to communicate with another device.

Computer 90 is connected to camera 210, microphone 220, an odor sensor 230, and button units 240 and 250. Camera 210 captures an image of a space in which a task is performed, and transmits the image to computer 90. Microphone 220 obtains audio in the space in which the task is performed, and transmits the audio to computer 90. Odor sensor 230 obtains a composition of air in the space in which the task is performed, and transmits the composition of the air to computer 90. Button unit 240 detects an operation done to button 241 or button 242, and transmits a detection result to computer 90. Button unit 250 detects an operation done to button 251 or button 252, and transmits a detection result to computer 90.

### [Overview of Processing]

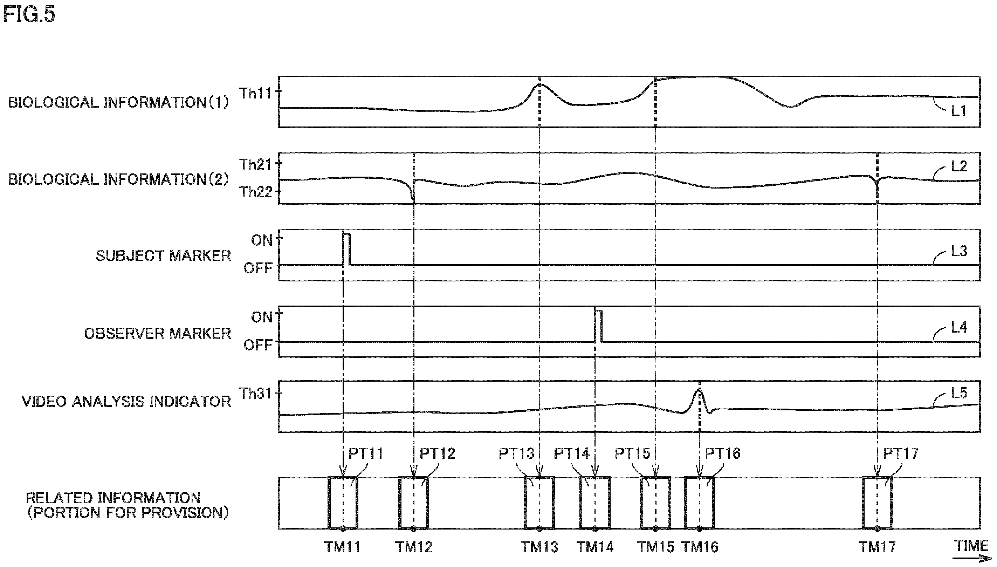

Fig. 5 generally represents an example of processing implemented in evaluation system 100.

Fig. 5 shows six types of data during a period of time in which subject 200 is performing a task. The six types of data include feature values of two types of biological information (biological information (1) and (2)), states (ON/OFF) of a subject marker and an observer marker, a video analysis indicator, and related information.

The biological information (1) represents, for example, a myoelectric potential signal of a muscle in a vicinity of a cheek. How the feature value of the biological information (1) changes is represented by a line L1. The biological information (2) represents, for example, a myoelectric potential signal of a muscle in a vicinity of an eyebrow. How the feature value of the biological information (2) changes is represented by a line L2.

The subject marker represents, for example, a state of operation of buttons 241 and 242. When button 241 is operated, the subject marker has a value turned ON, and when button 242 is operated, the subject marker has a value turned OFF. How the subject marker changes is represented by a line L3.

The observer marker represents, for example, a state of operation of buttons 251 and 252. When button 251 is operated, the observer marker has a value turned ON, and when button 252 is operated, the observer marker has a value turned OFF. How the observer marker changes is represented by a line L4.

The video analysis indicator represents a feature value obtained from an image captured with camera 210. One example of the feature value is a distance by which at least a part of the body of subject 200 moves within a predetermined unit period of time. The video analysis indicator is affected by movement of the body of subject 200 in the captured image. In this sense, it can be said that the video analysis indicator is one example of a feature value of biological information of subject 200. How the video analysis indicator changes is represented by a line L5.

The related information represents information obtained as information about a task. One example of the related information is an image captured with camera 210.

Computer 90 provides subject 200 with a portion of the related information that corresponds to a given timing. In the example of Fig. 5, seven timings (TM11 to TM17) are indicated as timings to determine portions to be provided.

More specifically, computer 90 determines a timing at which the value of the subject marker is turned ON as TM11.

Computer 90 determines a timing at which the value of the biological information (2) reaches a given threshold value (Th22) as TM12.

Computer 90 determines a timing at which the value of the biological information (1) reaches a given threshold value (Th11) (or decreases to the given threshold value) as TM13.

Computer 90 determines a timing at which the value of the observer marker is tuned ON as TM14.

Computer 90 determines a timing at which the value of the biological information (1) reaches the given threshold value (Th11) as TM15.

Computer 90 determines a timing at which the value of the video analysis indicator reaches a given threshold value (Th13) as TM16.

Computer 90 determines a timing at which the value of the biological information (2) reaches the given threshold value (Th22) (or decreases to the given threshold value) as TM17.

Computer 90 determines a period of time including a fixed period of time before and after each of the seven determined timings as a period of time for information provided to subject 200. Periods of time PT11 to PT17 are thus determined for timings TM11 to TM17, respectively. After the task ends, computer 90 provides subject 200 with respective portions of the related information for periods of time PT11 to PT17. The "fixed period of time" is, for example, 2 seconds. In that case, a motion video of 4 seconds is provided to subject 200 as a portion for each of periods of time PT11 to PT17.

### <When Task Related Information Is Provided>

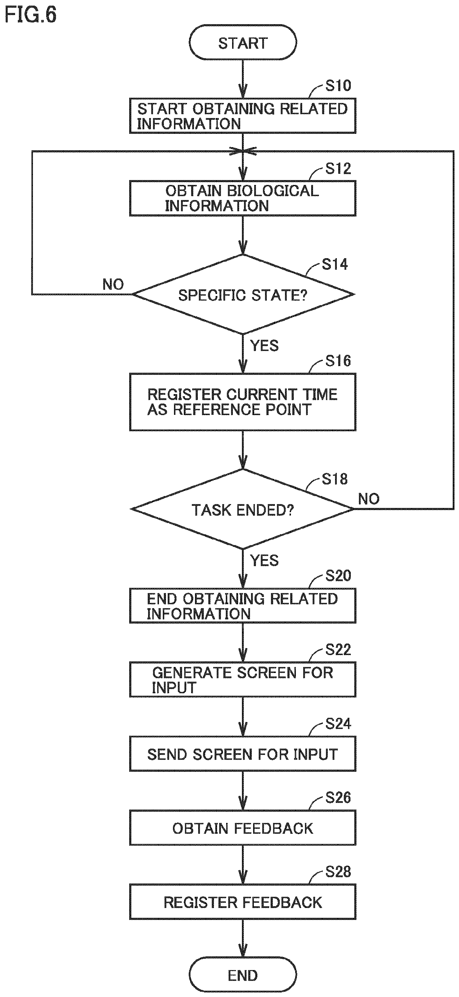

In the process described with reference to Fig. 6, after a task ends, a screen for input including such a portion as described above is generated and provided to subject 200. The screen for input may not be provided after the task ends. Computer 90 may generate and provide a screen for input to subject 200 whenever such a timing as described above is determined. That is, when timing TM1 is determined, computer 90 may provide the related information for period of time PT11 to subject 200 without waiting for the computer to determine the next timing TM2.

### <Period of Time to Which Information Provided Corresponds>

A period of time to which such a portion as described above corresponds is not required to be a period of time including a fixed period of time before and after a determined timing. That is, the period of time to which such a portion as described above corresponds may be a fixed period of time before and after the determined timing, the determined timing and a fixed period of time immediately therebefore, or the determined timing alone. For example, when a timing at which the biological information (1) reaches the given threshold value is determined, a video for the fixed period of time before and after the timing, a video for the timing and the fixed period of time immediately therebefore, or an image for the timing is provided to subject 200.

### <Method for Determining "Timing" and Types of Biological Information>

Instead of or in addition to the fact that a detected value of the biological information or a value of the video analysis indicator reaches a given threshold value, computer 90 may determine such a timing as described above by the fact that the detected value of the biological information or the value of the video analysis indicator is saturated (or the fact that an amount by which the value changes is equal to or smaller than a given value for a given period of time or longer). In this sense, the detected value of the biological information and the value of the video analysis indicator are each an example of a feature value of the biological information. Further, the fact that the value is saturated an example of the fact that the value is maintained within a given range.

It should be noted that the above-described "reaching a threshold value" may be either reaching the threshold value from below or reaching the threshold value from above (that is, decreasing toward the threshold value).

In the example of Fig. 5, a myoelectric potential signal of a muscle in a vicinity of a cheek, a myoelectric potential signal of a muscle in a vicinity of an eyebrow, and a video analysis indicator determined from a captured image of subject 200 are indicated as a specific example of a feature value of biological information. The feature value of the biological information may be other types of information such as a blood pressure value, a brain wave, a cardiac potential, a respiratory rate, and/or a heart rate of subject 200. For example, for the brain wave, a matching rate between a pattern of a brain wave of the subject and a predetermined waveform pattern may be used as an example of the feature value. That is, any information reflecting a state (such as emotion, etc.) of subject 200 can be used as the feature value of the biological information. And in evaluation system 100, computer 90 is configured to not only determine a timing based on the biological information but also determine a timing based on subject 200 or observer 300 operating a button. Computer 90 can thus also provide subject 200 with information corresponding to a timing which subject 200 or observer 300 determines to be potentially assistive for feedback.

### <Related Information (Task Related Information)>

Information obtained as the related information may be information of an ambience of the space in which the task is performed. The information of the ambience of the space is, for example, an image (or video) captured with camera 210, audio obtained through microphone 220, and/or information specifying a composition of air obtained through odor sensor 230. Camera 210 may capture an image of a field of view of subject 200 as the information of the ambience of the space. In that case, camera 210 is mounted on the head of subject 200 in order to capture the field of view of subject 200.

### <Mode of Obtaining Related Information>

The related information may be obtained continuously, or may be obtained whenever a determined period of time elapses or a given event occurs. In one example, as the related information, camera 210 may continuously capture a motion video while the task is performed. In another example, as the related information, camera 210 may capture an image (or take a photograph) whenever a determined period of time elapses while the task is performed. In yet another example, as the related information, camera 210 may capture an image (or take a photograph) whenever subject 200 presses button 241 and/or whenever observer 300 presses button 251.

### <Information Provided as Portion of Related Information>

Information provided as a portion of the related information (or portions corresponding to periods of time PT11 to PT17) may be a portion of the above-described related information per se, or may be information reproduced based on information determined from a portion of the related information. That is, information provided to subject 200 may be information secondarily generated based on the related information. For example, when the related information is in the form of image, and it is determined that an image for a determined timing includes a "peach," subject 200 may be provided, as information corresponding to that timing, with an image per se including the "peach," an audio representing "peach," air (or an aroma) having a composition predetermined to correspond to a "peach," a taste having a composition predetermined to correspond to a "peach," or a feel predetermined to correspond to a "peach".

### <Task>

In evaluation system 100, subject 200 performs a task and inputs feedback on the task. Computer 90 obtains the feedback from subject 200. The task performed by subject 200 is not limited to a task of assembling a shelf as described with reference to Fig. 1, and may be any type of task.

Another example of the task is a task for subject 200 to play a video game. When subject 200 plays the video game as the task, an image of a field of view of subject 200 as an example of the related information may be an image of the video game rather than captured with camera 210. The image of the video game may also be determined as the game proceeds. Accordingly, as the related information corresponding to a timing at which the biological information satisfies a given condition, computer 90 may obtain the progress of the game, rather than an image per se for such a timing.

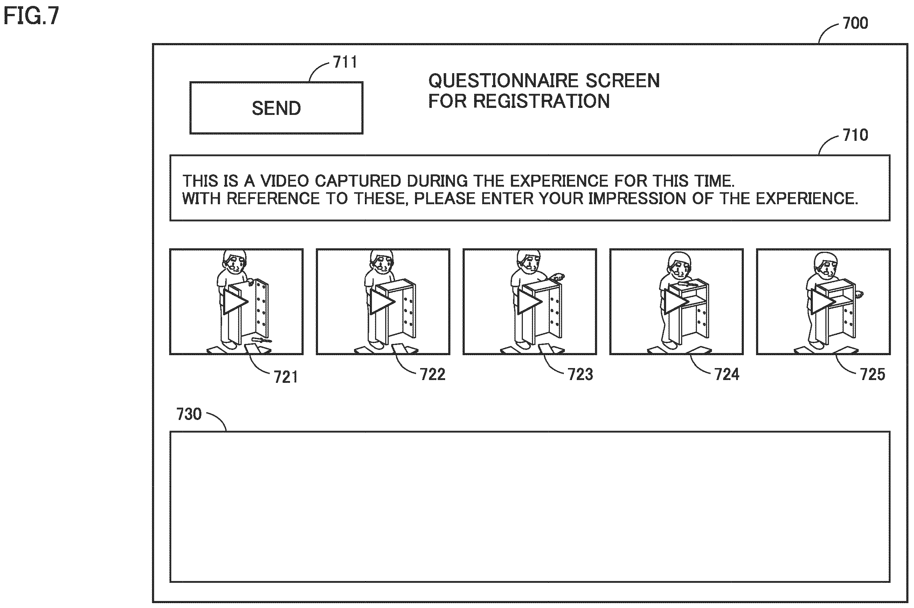

When generating a screen for input, as will be described hereinafter with reference to Fig. 7 and the like, computer 90 may reconstruct a screen based on the progress of the game. Then, computer 90 may provide the reconstructed screen to subject 200 by incorporating the reconstructed screen into the screen for input.

### [Process Flow]

Fig. 6 is a flowchart of a process performed by computer 90 to obtain feedback from subject 200 performing a task. Computer 90 starts the process of Fig. 6, for example, in response to notification input to computer 90 indicating that subject 200 starts a task. In one implementation, the process of Fig. 6 is performed in computer 90 by processor 95 executing a given program. The program may be stored in memory 96 in a non-transitory manner.

In step S10, computer 90 starts obtaining the related information. In one implementation, computer 90 instructs camera 210 to start recording.

In step S12, computer 90 obtains the biological information. In one implementation, computer 90 requests user device 10 to send a myoelectric potential signal obtained by sensor unit 1. In response to the request, user device 10 sends the myoelectric potential signal to computer 90.

In step S14, computer 90 determines whether a specific state has occurred. The specific state is a state in which the biological information satisfies a given condition, such as timing TM13 or TM15 determined in Fig. 5 for the biological information (1). When computer 90 determines that the specific state has occurred (YES in step S14), the control proceeds to step S16, otherwise (NO in step S14) the control returns to step S12.

In step S16, computer 90 registers the current time as a reference point in memory 96. Instead of the current time, a time having elapsed since the task was started may be registered.

In step S18, computer 90 determines whether the task ends. In one implementation, when the task ends, subject 200 operates button 242. When detecting that button 242 has been operated, computer 90 determines that the task has ended. When computer 90 determines that the task has ended (YES in step S18), the control proceeds to step S20, otherwise (NO in step S18) the control returns to step S12.

In step S20, computer 90 ends the obtaining the related information started in step S10. In one implementation, computer 90 instructs camera 210 to stop imaging.

In step S22, computer 90 generates a screen for input. The screen for input is a screen provided to subject 200 for inputting feedback.

Fig. 7 is a diagram for illustrating an example of a screen for input. A screen 700 includes a box 710, a key 711, boxes 721 to 725, and a box 730.

Box 710 includes a message "This is a video captured during the experience for this time. With reference to these, please enter your impression of the experience.". The "video captured during the experience for this time" indicates motion videos displayed in boxes 721 to 725, respectively, which will be described hereinafter. That is, the message in box 710 is an example of a message that urges subject 200 to refer to the provided video for inputting feedback. Computer 90 inserts the message into box 710 to generate a screen for input. The provided video is an example of a portion of the related information obtained while the task is performed, that corresponds to a "timing" described with reference to Fig. 5.

Key 711 is operated to send information input into a box 730, which will be described hereinafter, to computer 90.

Boxes 721 to 725 each display a motion video. The displayed motion video is a portion of a motion video (an example of the related information) obtained while the task is performed, that corresponds to a "timing" described with reference to Fig. 5. The "timing" is obtained as a "reference point" in the process of Fig. 6. In each of boxes 721 to 725, reproduction of a motion video is started in response to a touch operation.

Computer 90 cuts out of the motion video (an example of the related information) obtained while the task is performed the portions respectively corresponding to the "timings" described above to generate contents to be provided as "portions" corresponding to the "timings". Computer 90 inserts the generated contents into boxes 721 to 725, respectively, to generate a screen for input.

Boxes included in a screen for input for displaying related information such as a motion video is not limited to "5" boxes as shown in Fig. 7. In one implementation, the number of boxes is equal to the number of "timings" determined while the task is performed.

Box 730 receives feedback input by subject 200. Returning to Fig. 6, after generating a screen for input in step S22, computer 90 sends the screen for input to subject 200 in step S24. As described above, the screen for input has incorporated therein a portion of the related information corresponding to a "timing" described with reference to Fig. 5. In this sense, computer 90 sends the screen for input to subject 200 to provide the above-described portion to subject 200.

In one implementation, when a task starts, computer 90 obtains a mail address of subject 200 together with information which identifies subject 200. In step S24, computer 90 sends to the mail address a mail including such a screen for input that is generated as described above. Subject 200 receives the mail on smartphone 290 (Fig. 2). Thus, subject 200 views the screen for input on smartphone 290. Note that what is described herein is only one manner of sending a screen for input to subject 200. The screen for input may be sent to subject 200 in any manner.

In step S26, computer 90 obtains feedback from subject 200. In one implementation, subject 200 inputs feedback in text in box 730 on screen 700 displayed on smartphone 290, and thereafter operates key 711. In response, smartphone 290 sends the text input to box 730 to computer 90. Computer 90 receives the thus sent text and thus obtains feedback.

In step S28, computer 90 registers the obtained feedback in memory 96. Thereafter, computer 90 ends the process of Fig. 6.

Through the process described above, computer 90 provides subject 200 with a portion of the related information obtained while the task is performed, that corresponds to a timing determined using biological information of subject 200. Subject 200 inputs feedback on the task while referring to the portion, and sends the feedback to computer 90. Computer 90 thus obtains the feedback on the task from subject 200.

The process of Fig. 6 allows subject 200 to input feedback after the subject is provided with a portion of the information about the task (or the related information) that corresponds to a timing at which biological information of subject 200 satisfies a given condition. This allows subject 200 to more specifically remember a state that subject 200 per se had at the timing. This allows computer 90 to obtain more specific feedback from subject 200, and the obtained feedback reflects a true impression of the subject with high accuracy.

### [Modification]

### <Providing "Portion" of Related Information without Waiting for End of Task>

In the process of Fig. 6, screen 700 including boxes 721 to 725 is provided to subject 200 after the subject ends a task. Boxes 721 to 725 each display a portion of a motion video (an example of the related information) obtained while the task is performed, that corresponds to a "timing" described with reference to Fig. 5. That is, in the process of Fig. 6, two or more portions are collectively provided to subject 200 after the subject ends the task.

In contrast, the two or more portions may each be provided to subject 200 without waiting for the end of the task. For example, when a "timing" as described above is determined, computer 90 extracts from the related information a portion corresponding to that "timing" and sends the extracted portion to subject 200 without waiting for the end of the task. Subject 200 can continue the task while being aware of a situation in which biological information of subject 200 satisfies a given condition, even while the subject performs a task, and it is expected that what should be input as feedback can be easily output.

### <Number of "Portions" Of Related Information Included in Single Screen for Input>

The Fig. 7 screen 700 collectively provides subject 200 with a plurality of portions corresponding to a plurality of timings as five boxes 721 to 725. Note that the screen for input may present a plurality of portions respectively corresponding to a plurality of timings to subject 200 one by one, and may display one box for one portion for feedback input. That is, for a task, when five portions are provided to subject 200, five screens are provided for input. The five screens for input each include one box for displaying the above-described portion and one box for inputting feedback while referring to that portion.

### <Outputting State of Subject>

In generating a screen for input, computer 90 may analyze content of the above-described "portion" provided to subject 200 to determine a state of subject 200 and may include the determined state in the screen for input.

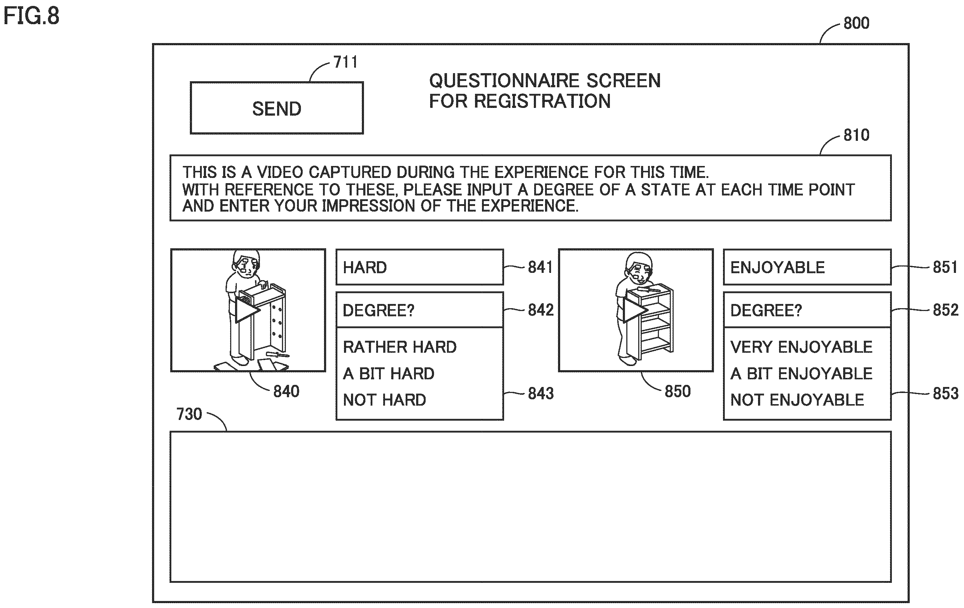

Fig. 8 is a diagram showing another example of the screen for input. As well as the Fig. 7 screen 700, a screen 800 includes box 730 and key 711.

Screen 800 does not include box 710 of screen 700 and instead includes a box 810. Box 810 displays a message, which includes the message displayed in box 710, and in addition, a phrase "(Please) input a degree of a state at each time point". This phrase urges subject 200 to input degrees of states that subject 200 has at timings when motion videos displayed in boxes 840 and 850 described hereinafter are captured.

Screen 800 includes boxes 840 and 850. Boxes 840 and 850 each display a motion video in the same manner as each of boxes 721 to 725 shown in Fig. 7. The displayed motion video is a portion of a motion video (an example of the related information) obtained while the task is performed, that corresponds to a "timing" described with reference to Fig. 5.

Screen 800 includes fields 841 and 851. Fields 841 and 851 display states of subject 200 determined from the motion videos displayed in boxes 840 and 850, respectively. In one implementation, computer 90 extracts a specific frame (e.g., an initially displayed frame, a frame displayed at an intermediate point in time, etc.) from the motion video displayed in box 840, determines a facial region from an image in the frame, and analyzes the image of the determined facial region to determine an emotion corresponding to the facial image. Then, computer 90 embeds the determined emotion in field 841 as a state of subject 200. Similarly, computer 90 determines an emotion of subject 200 from box 850, and embeds the emotion in field 851 as a state of subject 200.

Screen 800 includes fields 842 and 843. Field 842 is a field for inputting a degree of a state displayed in field 841. Field 843 is a pull-down menu and displays candidates for the degree input to field 842. In the example of Fig. 8, field 841 displays a state "hard". Field 843 displays three candidates, that is, "rather hard," "a bit hard," and "not hard" for degrees for the state "hard". Subject 200 selects one of the candidates displayed in field 843. The selected candidate is input to field 842.

Screen 800 includes fields 852 and 853. Field 852 is a field for inputting a degree of a state displayed in field 851. Field 853 is a pull-down menu and displays candidates for the degree of input to field 852. In the example of Fig. 8, field 851 displays a state "enjoyable". Field 853 displays three candidates, that is, "very enjoyable," "a bit enjoyable," and "not enjoyable," for degrees for the state "enjoyable". Subject 200 selects one of the candidates displayed in field 853. The selected candidate is input to field 852.

On screen 800 key 711 is pressed to send to computer 90 the text input to box 730 and in addition thereto the degrees of the states input to fields 842 and 852. Thus, in step S26, computer 90 obtains, as feedback, the text input to box 730 and in addition thereto the degrees of the states input to fields 842 and 852.

In the above-described embodiment, an example in which a single computer 90 performs each step has been described. The present invention is not limited thereto, and a plurality of communicably connected computers may each perform a different step.

### [Aspects]

It will be understood by those skilled in the art that a plurality of exemplary embodiments described above are specific examples of the following aspects.

(Clause 1) In one aspect, an evaluation method may comprise: obtaining information about a task performed by a subject; obtaining biological information of the subject performing the task over time; determining a timing at which the biological information satisfies a predetermined condition; outputting a portion of the information about the task corresponding to the determined timing; and receiving an input of feedback on the task from the subject.

The evaluation method according to clause 1 allows more specific feedback to be obtained from the subject in evaluating a response of the subject.

(Clause 2) The evaluation method according to clause 1, wherein the biological information may include at least one of: a myoelectric potential signal of a facial muscle of the subject; a blood pressure value of the subject; a brain wave of the subject; a cardiac potential of the subject; a respiratory rate of the subject; a heart rate of the subject; and an indicator determined from an image including the subject.

The evaluation method according to clause 2 allows a state of the subject to be accurately reflected in the biological information.

(Clause 3) The evaluation method according to clause 1 or 2, wherein the predetermined condition may include at least one of: that a feature value in the biological information reaches a given threshold value; and that an amount by which the feature value in the biological information changes continues to have a given value or smaller for a prescribed period of time or longer.

The evaluation method according to clause 3 facilitates determining whether a feature value of the biological information satisfies a predetermined condition.

(Clause 4) The evaluation method according to any one of clauses 1 to 3, wherein the predetermined condition may include that a feature value in the biological information is maintained within a given range for a given period of time or longer.

The evaluation method according to clause 4 facilitates determining whether a feature value of the biological information satisfies a predetermined condition.

(Clause 5) The evaluation method according to any one of clauses 1 to 4 may further comprise receiving an input of a marker from at least one of the subject and an observer observing the subject while the subject performs a task, wherein the outputting may include providing a portion of the information about the task corresponding to a time point at which the marker is put.

The evaluation method according to clause 5 can also provide the subject with information for a portion corresponding to a timing desired by the subject or an observer.

(Clause 6) In one aspect, an evaluation method may comprise: obtaining information about a task performed by a subject; determining a timing at which an input of a marker is received from at least one of the subject performing the task and an observer observing the subject while the subject performs the task; outputting a portion of the information about the task corresponding to the determined timing; and receiving an input of feedback on the task from the subject.

The evaluation method according to clause 6 allows more specific feedback to be obtained from the subject in evaluating a response of the subject.

(Clause 7) The evaluation method according to any one of clauses 1 to 6, wherein the information about the task may include at least one of: information of an ambience of a space in which the task is performed; and information of content provided to the subject in the task.

The evaluation method according to clause 7 allows a state of the subject to be accurately reflected in the information about the task.

(Clause 8) The evaluation method according to clause 7, wherein the information about the task may include the information of the ambience of the space, and the information of the ambience of the space may include at least one of: a captured image of the space in which the task is performed; recording of audio in the space in which the task is performed; and an odor of the space in which the task is performed.

The evaluation method according to clause 8 allows a state of the subject to be more accurately reflected in the information about the task.

(Clause 9) The evaluation method according to clause 7, wherein the information about the task may include the information of the ambience of the space, the information of the ambience of the space may include a captured image of the space, and the captured image of the space may include at least one of: an image including the subject performing the task; and an image according to a line of sight of the subject performing the task.

The evaluation method according to clause 9 allows a state of the subject to be further accurately reflected in the information about the task.

(Clause 10) The evaluation method according to any one of clauses 7 to 9, wherein the information about the task may include the information of the content, and the portion output may include at least one of an image, an audio, an odor, a feel, and a taste.

The evaluation method according to clause 10 can provide the subject with information useful for inputting specific feedback.

(Clause 11) The evaluation method according to any one of clauses 1 to 10, wherein the obtaining may include obtaining the information about the task whenever a determined period of time elapses while the task is performed.

The evaluation method according to clause 11 can minimize an amount of data handled in obtaining the information about the task.

(Clause 12) The evaluation method according to any one of clauses 1 to 11, wherein the portion output may correspond to the timing and a given period of time before and after the timing.

The evaluation method according to clause 12 can provide the subject with detailed information for reminding the subject of a situation at the timing.

(Clause 13) The evaluation method according to any one of clauses 1 to 12, wherein the outputting may include providing the portion after the task ends.

The evaluation method according to clause 13 can remind the subject of a situation while the task is performed even after the task ends.

(Clause 14) The evaluation method according to any one of clauses 1 to 13, wherein the outputting may include providing the portion in response to the timing being determined without waiting for the task to end.

The evaluation method according to clause 14 can more reliably remind the subject of a state that the subject has at the timing.

(Clause 15) The evaluation method according to any one of clauses 1 to 14, wherein the outputting may include: determining a state of the subject by analyzing the portion output; and outputting the state of the subject.

The evaluation method according to clause 15 can provide the subject with more specific information about the subject.

(Clause 16) The evaluation method according to clause 15, wherein the outputting may include outputting information urging that a degree of the state of the subject be input, and the receiving may include obtaining an input of the degree of the state of the subject.

The evaluation method according to clause 16 allows a degree of a state of the subject to be managed in a computer performing the evaluation method.

(Clause 17) The evaluation method according to any one of clauses 1 to 16, wherein the outputting may include, when a first timing and a second timing are determined as the timing, providing a portion corresponding to the first timing, and together therewith, a portion corresponding to the second timing.

The evaluation method according to clause 17 can provide the subject with information comprehensively.

(Clause 18) The evaluation method according to any one of clauses 1 to 17, wherein the outputting may include, when a first timing and a second timing are determined as the timing, providing a portion corresponding to the first timing and thereafter providing a portion corresponding to the second timing.

The evaluation method according to clause 18 provides the subject with information about a first timing and information about a second timing individually.

(Clause 19) The evaluation method according to any one of clauses 1 to 18, wherein the outputting may include outputting a message to urge the subject to refer to the output portion in inputting feedback.

The evaluation method according to clause 19 ensures causing the subject to refer to the information of the portion.

(Clause 20) In one aspect, an evaluation apparatus may comprise: an interface that obtains information about a task performed by a subject and biometric information of the subject; a memory that stores information that specifies a condition for a feature value in the biological information; and a processor that determines a timing at which the feature value in the biological information satisfies the condition, wherein the processor may output a portion of the information about the task corresponding to the determined timing, and receive an input of feedback on the task.

The evaluation apparatus according to clause 20 can obtain more specific feedback from the subject in evaluating a response of the subject.

(Clause 21) In one aspect, an evaluation apparatus may comprise: an interface that obtains information about a task performed by a subject and biometric information of the subject; and a processor that determines a timing at which an input of a marker is received from at least one of the subject performing the task and an observer observing the subject while the subject performs the task, wherein the processor may output a portion of the information about the task corresponding to the determined timing, and receive an input of feedback on the task.

The evaluation apparatus according to clause 21 can obtain more specific feedback from the subject in evaluating a response of the subject.

(Clause 22) In one aspect, a program may be executed by a computer to cause the computer to perform: obtaining information about a task performed by a subject; obtaining biological information of the subject; determining a timing at which the biological information satisfies a predetermined condition; outputting a portion of the information about the task corresponding to the determined timing; and receiving an input of feedback on the task.

The program according to clause 22 allows more specific feedback to be obtained from the subject in evaluating a response of the subject.

(Clause 23) In one aspect, a program may be executed by a computer to cause the computer to perform: obtaining information about a task performed by a subject; determining a timing at which an input of a marker is received from at least one of the subject performing the task and an observer observing the subject while the subject performs the task; outputting a portion of the information about the task corresponding to the determined timing; and receiving an input of feedback on the task from the subject.

The program according to clause 23 allows more specific feedback to be obtained from the subject in evaluating a response of the subject.

It should be understood that the presently disclosed embodiments are illustrative and non-restrictive in any respect. The scope of the present disclosure is defined by the terms of the claims, rather than the above description of the embodiments, and is intended to encompass any modifications within the meaning and scope equivalent to the terms of the claims. Furthermore, each technique in the embodiments is intended to be used alone or in combination with other techniques in the embodiments as much as possible, as necessary.

### REFERENCE SIGNS LIST

1 sensor unit, 3, 91 controller, 4, 92 communication module, 5 battery, 6 housing, 10 user device, 11 first myoelectric potential sensor, 12 second myoelectric potential sensor, 21 speaker, 22 display, 23 content provision device, 31, 95 processor, 32, 96 memory, 33, 97 input/output port, 90 computer, 100 evaluation system, 111, 121 working electrode, 112, 122 reference electrode, 151, 152, 171, 172 pad, 200 subject, 210 camera, 220 microphone, 230 odor sensor, 240, 250 button unit, 241, 242, 251, 252 button, 290 smartphone, 300 observer, 700, 800 screen, 710, 721, 725, 730, 810, 840, 850 box, 711 key, 841, 842, 843, 851, 852, 853 field.

## Claims

1. An evaluation method comprising:
obtaining information about a task performed by a subject;
obtaining biological information of the subject performing the task over time;
determining a timing at which the biological information satisfies a predetermined condition;
outputting a portion of the information about the task corresponding to the determined timing; and
receiving an input of feedback on the task from the subject.

2. The evaluation method according to claim 1, wherein the biological information includes at least one of:
a myoelectric potential signal of a facial muscle of the subject;
a blood pressure value of the subject;
a brain wave of the subject;
a cardiac potential of the subject;
a respiratory rate of the subject;
a heart rate of the subject; and
an indicator determined from an image including the subject.

3. The evaluation method according to claim 1, wherein the predetermined condition includes at least one of:
that a feature value in the biological information reaches a given threshold value; and
that an amount by which the feature value in the biological information changes continues to have a given value or smaller for a prescribed period of time or longer.

4. The evaluation method according to claim 1, wherein the predetermined condition includes that a feature value in the biological information is maintained within a given range for a given period of time or longer.

5. The evaluation method according to claim 1, further comprising receiving an input of a marker from at least one of the subject and an observer observing the subject while the subject performs a task, wherein
the outputting includes providing a portion of the information about the task corresponding to a time point at which the marker is put.

6. An evaluation method comprising:
obtaining information about a task performed by a subject;
determining, while the subject performs the task, a timing at which an input of a marker is received from at least one of the subject performing the task and an observer observing the subject;
outputting a portion of the information about the task corresponding to the determined timing; and
receiving an input of feedback on the task from the subject.

7. The evaluation method according to claim 1, wherein the information about the task includes at least one of:
information of an ambience of a space in which the task is performed; and
information of content provided to the subject in the task.

8. The evaluation method according to claim 7, wherein
the information about the task includes the information of the ambience of the space, and
the information of the ambience of the space includes at least one of:
a captured image of the space;
recording of audio in the space; and
an odor of the space.

9. The evaluation method according to claim 7, wherein
the information about the task includes the information of the ambience of the space,
the information of the ambience of the space includes a captured image of the space, and
the captured image of the space includes at least one of:
an image including the subject performing the task; and
an image according to a line of sight of the subject performing the task.

10. The evaluation method according to claim 7, wherein
the information about the task includes the information of the content, and the portion output includes at least one of an image, an audio, an odor, a feel, and a taste.

11. The evaluation method according to claim 1, wherein the obtaining includes obtaining the information about the task whenever a determined period of time elapses while the task is performed.

12. The evaluation method according to claim 1, wherein the portion output corresponds to the timing and a given period of time before and after the timing.

13. The evaluation method according to claim 1, wherein the outputting includes outputting the portion after the task ends.

14. The evaluation method according to claim 1, wherein the outputting includes outputting the portion in response to the timing being determined without waiting for the task to end.

15. The evaluation method according to claim 1, wherein the outputting includes:
determining a state of the subject by analyzing the portion output; and
outputting the state of the subject.

16. The evaluation method according to claim 15, wherein
the outputting includes outputting information urging that a degree of the state of the subject be input, and
the receiving includes obtaining an input of the degree of the state of the subject.

17. The evaluation method according to claim 1, wherein the outputting includes, when a first timing and a second timing are determined as the timing, providing a portion corresponding to the first timing, and together therewith, a portion corresponding to the second timing.

18. The evaluation method according to claim 1, wherein the outputting includes, when a first timing and a second timing are determined as the timing, outputting a portion corresponding to the first timing and thereafter providing a portion corresponding to the second timing.

19. The evaluation method according to claim 1, wherein the outputting includes outputting a message to urge the subject to refer to the output portion in inputting feedback.

20. An evaluation apparatus comprising:
an interface that obtains information about a task performed by a subject and biometric information of the subject;
a memory that stores information that specifies a condition for a feature value in the biological information; and
a processor that determines a timing at which the feature value in the biological information satisfies the condition, wherein
the processor outputs a portion of the information about the task corresponding to the determined timing, and receives an input of feedback on the task.

21. An evaluation apparatus comprising:
an interface that obtains information about a task performed by a subject and biometric information of the subject; and
a processor that determines a timing at which an input of a marker is received from at least one of the subject performing the task and an observer observing the subject while the subject performs the task, wherein
the processor outputs a portion of the information about the task corresponding to the determined timing, and receives an input of feedback on the task.

22. A program, when executed by a computer, causing the computer to perform:
obtaining information about a task performed by a subject;
obtaining biological information of the subject;
determining a timing at which the biological information satisfies a predetermined condition;
outputting a portion of the information about the task corresponding to the determined timing; and
receiving an input of feedback on the task.

23. A program, when executed by a computer, causing the computer to perform:
obtaining information about a task performed by a subject;
determining a timing at which an input of a marker is received from at least one of the subject performing the task and an observer observing the subject while the subject performs the task;
outputting a portion of the information about the task corresponding to the determined timing; and
receiving an input of feedback on the task from the subject.
